(19) Europäisches Patentamt
European Patent Office
Office européen des brevets

(11) **EP 2 180 862 B1**

(12) **EUROPÄISCHE PATENTSCHRIFT**

(45) Veröffentlichungstag und Bekanntmachung des Hinweises auf die Patenterteilung:
**04.01.2012 Patentblatt 2012/01**

(21) Anmeldenummer: **08851722.2**

(22) Anmeldetag: **06.11.2008**

(51) Int Cl.:
**A61F 13/496** (2006.01)

(86) Internationale Anmeldenummer:
**PCT/EP2008/009361**

(87) Internationale Veröffentlichungsnummer:
**WO 2009/065498 (28.05.2009 Gazette 2009/22)**

(54) **INKONTINZENZARTIKEL IN HÖSCHENFORM**

PANTY-TYPE INCONTINENCE ARTICLE

ARTICLE POUR L'INCONTINENCE SOUS FORME DE CULOTTE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.11.2007 DE 102007055628**

(43) Veröffentlichungstag der Anmeldung:
**05.05.2010 Patentblatt 2010/18**

(73) Patentinhaber: **Paul Hartmann Aktiengesellschaft 89522 Heidenheim (DE)**

(72) Erfinder:
• **HORNRUNG, Fridmann Santiago (CL)**

• **WENZEL, Benjamin 89231 Neu-Ulm (DE)**
• **OSTERTAG, Wolfgang 89547 Gerstetten (DE)**

(74) Vertreter: **Friz, Oliver Dreiss Patentanwälte Postfach 10 37 62 70032 Stuttgart (DE)**

(56) Entgegenhaltungen:
JP-A- 11 036 103    JP-A- 2000 014 700
JP-A- 2003 126 148    US-A1- 2006 173 436
US-B1- 6 497 696

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt und Rückenabschnitt erstreckt und an den Bauchabschnitt und an den Rückenabschnitt unlösbar angefügt ist, wobei sowohl der Schrittabschnitt als auch der Bauchabschnitt und der Rückenabschnitt die Beinöffnungen des Inkontinenzartikels begrenzen. Ein derart dreikomponentiger Inkontinenzartikel ist beispielsweise aus WO 2004/052260 A1 bekannt. Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der vorerwähnten WO 2004/052260 A1 oder JP 2003 126148 behandelt.

Im Zuge der Entwicklung von Inkontinenzartikeln in Höschenform wurde erkannt, dass möglichst weitgehende Bereiche des Inkontinenzartikels elastisch nachgiebig, also entsprechend einer Körperform des Benutzers dehnbar, ausgebildet werden sollten. Dies führte zu der nicht immer zutreffenden Annahme, dass ein größtmöglicher Teil der hüllenbildenden Chassismaterialien des Inkontinenzartikels mit elastisch dehnbaren oder elastifizierten Materialien, insbesondere durch Einbringen vorgenannter Elastifizierungsmittel im Stretch-Bond-Verfahren, ausgebildet werden sollten. Dessen ungeachtet bzw. zusätzlich war man bestrebt, die Beinöffnungen des Inkontinenzartikels möglichst durchgehend zu elastifizieren, um einen sicheren Seitenauslaufschutz zu erreichen.

Es wurde aber nicht erkannt, dass die elastische Ausbildung von Chassismaterialien, insbesondere durch Einbringen von linear erstreckten Elastifizierungsmitteln auch mit nicht unerheblichen Nachteilen verbunden ist. Zwar kann mit einer flächenhaften, also im Wesentlichen über die Erstreckung durchgehenden Elastifizierung von Chassismaterialien erreicht werden, dass sich der Inkontinenzartikel in Höschenform überhaupt in gewisser Weise an unterschiedliche Körperformen anpassen lässt, der Artikel also überhaupt in bestimmungsgemäßer Form an einem Benutzer positionierbar ist. Dies ist jedoch häufig mit der Ausbildung sehr starker Zugspannungen in den Chassismaterialien verbunden, die sich in unangenehmer Weise auf den Benutzer auswirken. Hierbei schneiden die Elastifizierungsmittel oftmals in die Hautoberfläche des Benutzers ein, was als unangenehm empfunden wird und sogar Schmerzen, Hautreizungen und, insbesondere in Verbindung mit einem humiden Klima, sogar Hautverletzungen erzeugen kann. Unter hoher Dehnungsspannung stehende Chassismaterialien liegen häufig eng gegen die Hautoberfläche des Benutzers an, was auch bei Verwendung atmungsaktiver Materialien zu einem feuchten Mikroklima im Bereich der Hautoberfläche führt und ebenfalls fatale Folgen im Hinblick auf die Hautoberfläche des Benutzers haben kann. Solchenfalls bildet sich unmittelbar angrenzend an die Hautoberfläche nämlich keine Gasphase sondern eine Flüssigkeitsphase aus. Ungeachtet dessen führt eine starke Vorspannung im Bereich der Elastifizierungsmittel zu starker Rüschenbildung, also zu einer großen Anzahl von Falten oder Fältelungen pro Zentimeter (betrachtet in Richtung der elastischen Wirkung der Elastifizierungsmittel). Diese dreidimensional gewellte Struktur wird dann unter großer Spannung, welche durch die Elastifizierungsmittel ausgeübt wird, gegen die Hautoberfläche gedrückt und verursacht insbesondere bei hoher Mobilität des Benutzers Relativbewegungen, die wiederum die Hautoberfläche reizen und zu unangenehmen bis hin zu medizinisch problematischen Hautreizungen führen.

Wann immer Elastifizierungsmittel in Bezug auf eine Maschinenrichtung bogenförmig oder quer geführt werden, was zur Erreichung einer flächenhaften, im Wesentlichen die gesamten Chassismaterialien erfassenden Elastifizierung häufig angewandt wird, tritt das Problem auf, dass in Folge der Komponente quer zur Maschinenrichtung ein größerer Weg zurückgelegt werden muss und damit eine stärkere Vorspannung der Elastifizierungsmittel im Zuge des Stretch-Bond-Verfahrens einhergeht. Dies führt typischerweise zu einem stärkeren elastischen Dehnungswiderstand der entsprechenden Chassisbereiche verglichen mit Bereichen, in denen die Elastifizierungsmittel in Maschinenrichtung verlaufend eingebracht sind, was sich wiederum in höchstem Maße problematisch auswirken kann.

Hiervon ausgehend liegt der vorliegenden Erfindung die Aufgabe zugrunde, einen Inkontinenzartikel der eingangs beschriebenen Art im Hinblick auf den Tragekomfort zu verbessern, und zwar gerade in vom Hüftrand des Inkontinenzartikels abgewandten, also eher schrittzugewandten Bereichen des Bauchabschnitts und des Rückenabschnitts, wo sich die vorausgehend geschilderten Probleme erfahrungsgemäß am kritischsten stellen.

Diese Aufgabe wird nach der Erfindung gelöst durch einen Inkontinenzartikel mit den Merkmalen des Anspruchs 1.

Dadurch, dass sowohl der Bauchabschnitt als auch der Rückenabschnitt mit einer in Richtung auf eine Quermittelachse zulaufenden Randkontur zur Begrenzung der Beinöffnungen ausgebildet sind, kann einerseits eine große flächenhafte Überdeckung zwischen Schrittabschnitt und Bauchabschnitt bzw. Schrittabschnitt und Rückenabschnitt erreicht werden, was für die Ausbildung einer stabilen Verbindung zwischen den drei Komponenten des Inkontinenzartikels wesentlich ist. Zum anderen wird hierdurch eine den Beinöffnungen entsprechende Konturlinie geschaffen. Des Weiteren wird ein in Richtung auf die Quermittelachse erstreckter schrittseitiger und den Beinöffnungen zugewandter Bereich des Bauchabschnitts und des Rückenabschnitts geschaffen, in dem die zweiten Elastifizierungsmittel spezifisch entsprechend der dort erwünschten Spannungsverhältnisse eingebracht werden können.

Zur flächenhaften Elastifizierung von Bauchabschnitt und Rückenabschnitt sind die jeweils in einem Abstand voneinander und parallel zueinander in Quer- oder Hüftumfangsrichtung erstreckten ersten Elastifizierungsmittel vorgesehen. Diese haben vorzugsweise gleiche Vorspannung und dienen im Wesentlichen einer flächenhaft durchgehenden gleichmäßigen Elastifizierung des Bauchabschnitts und des Rückenabschnitts in dem Bereich deutlich oberhalb der Beinöffnungen. Es ist indessen möglich, dass die ersten Elastifizierungsmittel in einem oberen Hüftrandbereich eine stärkere Vorspannung aufweisen oder mehrere dieser Elastifizierungsmittel mit geringerem Abstand voneinander vorgesehen sind, um eine etwas stärkere Elastifizierung am Hüftrand zu realisieren.

Es wurde mit der vorliegenden Erfindung unter anderem erkannt, dass die Spannungsverhältnisse in dem genannten schrittseitigen und den Beinöffnungen zugewandten Bereich des Bauchabschnitts und des Rückenabschnitts für die eingangs geschilderten Probleme verantwortlich sind und so gestaltet werden können, dass sich die genannten Probleme nicht oder in geringerem Maße stellen. Erfindungsgemäß ist der schrittseitige und den Beinöffnungen zugewandte Bereich, in welchem die zweiten Elastifizierungsmittel sich in Richtung auf die Längsmittelachse auffächern, so ausgebildet, dass die bei flächenhafter Dehnung dieses Bereichs auftretende Rückstellkraft geringer ist als bei flächenhafter Dehnung in einem hüftseitigen Bereich, in welchem nur die ersten Elastifizierungsmittel vorgesehen sind. Mit dem Begriff "hüftseitiger Bereich" ist gemeint, dass sich dieser Bereich in Längsrichtung außerhalb des schrittseitigen und den Beinöffnungen zugewandten Bereichs mit den sich auffächernden zweiten Elastifizierungsmitteln befindet. Es handelt sich bei der Rückstellkraft um diejenige Kraft, welche der Bauchabschnitt und der Rückenabschnitt einer flächenhaften Dehnung in Richtung des Verlaufs der Elastifizierungsmittel entgegensetzen. Es ist erfindungsgemäß als wesentlich erkannt worden, dass der schrittseitige und den Beinöffnungen zugewandte Bereich bei flächenhafter Dehnung entlang der zweiten Elastifizierungsmittel mit geringerer Rückstellkraft ausgebildet wird als daran in Längsrichtung angrenzende Bereiche, die weiter vom Schritt und den Beinöffnungen entfernt sind, insbesondere die Bereiche, in denen die ersten Elastifizierungsmittel angeordnet sind. Hierdurch wird ein besserer Tragekomfort des Inkontinenzartikels erreicht, und es kommt in geringerem Umfang zu den geschilderten Problemen, weil der Inkontinenzartikel im Bereich des Schritts bzw. im Bereich der Beinöffnungen der Körperform entsprechend auch in größerem Umfang gedehnt werden kann, ohne dass es gleich zu einer unangenehmen Erhöhung der Rückstellkräfte mit den eingangs geschilderten Konsequenzen kommt.

Zur Bestimmung der Rückstellkräfte können die zu vermessenden Bereiche des Chassis direkt, gleichsam zerstörungsfrei zwischen zwei Klemmbacken von definierter, gleicher Klemmbackenbreite fest eingespannt und die Rückstellkräfte bei einer definierten, den Gebrauchszustand simulierenden Dehnung der zu messenden Bereiche um insbesondere 30% oder 50% oder 80% der Ausgangslänge (des Klemmbackenabstands bei Fixieren des zu messendes Bereichs in ungespanntem Zustand) ermittelt werden. Die Klemmbacken sollten möglichst viele, zumindest jedoch zwei nebeneinander angeordnete Elastifizierungsmittel des zu messenden Bereiches fixieren und im Wesentlichen senkrecht zum Verlauf der Elastifizierungsmittel orientiert sein, so dass die Dehnung zwischen den Klemmen im Wesentlich in Richtung des Verlaufs der Elastifizierungsmittel erfolgt.

In Weiterbildung dieses Erfindungsgedankens erweist es sich als vorteilhaft, wenn die bei flächenhafter Dehnung dieses schrittseitigen und den Beinöffnungen zugewandten Bereichs auftretende Rückstellkraft zusätzlich innerhalb dieses Bereichs in Richtung auf den Schrittabschnitt bzw. eine Längsmittelachse des Inkontinenzartikels abnimmt. Betrachtet man also diesen schrittseitigen und den Beinöffnungen zugewandten Bereich des Bauchabschnitts und des Rückenabschnitts, und zwar in einer Richtung ausgehend von dem jeweiligen Seitennahtbereich in Richtung auf den Schrittabschnitt, also gewissermaßen in Richtung der bogenförmigen Auffächerung der zweiten Elastifizierungsmittel, so erweist es sich als vorteilhaft, wenn die bei flächenhafter Dehnung auftretende Rückstellkraft in dieser Richtung abnimmt. Es handelt sich hier wiederum um diejenige Kraft, welche der Bauchabschnitt und der Rückenabschnitt einer flächenhaften Dehnung entgegensetzen. Eine Abnahme dieser Rückstellkraft in der genannten Richtung ist mit einer weiteren erheblichen Verbesserung des Tragekomforts des Inkontinenzartikels verbunden, und zwar in besonderem Maße im Gesäßbereich des Inkontinenzartikels.

Es erweist sich weiter als besonders vorteilhaft, wenn die Abnahme der Rückstellkraft in dem genannten schrittseitigen und den Beinöffnungen zugewandten Bereich des Bauchabschnitts und des Rückenabschnitts so vorgesehen wird, dass in Richtung auf den Schrittabschnitt eine abnehmende Anzahl von Falten pro cm-Querrichtung des Inkontinenzartikels gebildet wird. Solchenfalls können sich der Bauchabschnitt und der Rückenabschnitt entsprechend der Körperform des Benutzers dehnen, ohne dass die hierdurch gebildeten elastischen Kräfte das Chassismaterial mit einer Vielzahl von Falten zu raffen versuchen. Es sei an dieser Stelle nochmals erläutert, dass die Abnahme der Rückstellkraft in Richtung auf den Schrittabschnitt bedeutet, dass diejenige Kraft, die infolge einer flächenhaften Dehnung erzeugt wird, mit zunehmender Annäherung an den Schrittabschnitt geringer wird. Die Rückstellkraft infolge flächenhafter Dehnung ist also in einem Gebiet näher der Seitennaht größer als in einem Gebiet näher dem Schrittabschnitt.

Die genannten Spannungsverhältnisse lassen sich in verschiedenster Weise erreichen, etwa durch Verwendung von in Querrichtung unterschiedlich elastischen Materialien in dem schrittseitigen und den Beinöffnungen zugewandten Bereich, in welchem auch die zweiten Elastifizierungsmittel vorgesehen sind. Es wäre auch denkbar, dass die Vorspannung der zweiten Elastifizierungsmittel mit zunehmender Annäherung an den Schrittabschnitt, also von außen nach innen in Richtung auf eine Längsmittelachse reduziert wird. Außerdem wäre es denkbar, dass die Abnahme der Rückstellkraft bei flächenhafter Dehnung dadurch erreicht wird, dass der Abstand der zweiten Elastifizierungsmittel voneinander zunimmt, wobei hier darauf zu achten ist, dass dies nicht mit einer starken Zunahme der Vorspannung infolge des fächerförmigen Verlaufs der Elastifizierungsmittel ausgeglichen oder gar in Richtung zunehmender Rückstellkraft übertroffen wird.

Es hat sich insbesondere als vorteilhaft erwiesen, wenn ein minimaler Abstand der zweiten Elastifizierungsmittel voneinander (Abstand von unmittelbar nebeneinander liegenden Elastifizierungsmitteln) in den Seitennahtbereichen 3 bis 8 mm, insbesondere 3 bis 7 mm und weiter insbesondere 3 bis 6 mm beträgt.

Des Weiteren hat es sich als vorteilhaft erwiesen, wenn ein maximaler Abstand der zweiten Elastifizierungsmittel voneinander (Abstand von unmittelbar nebeneinander liegenden Elastifizierungsmitteln) an einem Absorptionskörperrand oder an einem Längsrand des Schrittabschnitts 7 bis 35 mm, insbesondere 10 bis 32 mm und weiter insbesondere 12 bis 30 mm beträgt.

Des Weiteren hat es sich als vorteilhaft erwiesen, wenn die zweiten Elastifizierungsmittel einen Auffächerungsgrad F

$$F = (A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am Längsrand des Schrittabschnitts oder am Absorptionskörperrand, und B als der minimale Abstand insbesondere im Seitennahtbereich. Es wurde auch erkannt, dass es sich als vorteilhaft erweist, wenn der Auffächerungsgrad F der zweiten Elastifizierungsmittel im Rückenabschnitt größer ist als im Bauchabschnitt.

Aufgrund der naturbedingten Körperformen im Rückenbereich bzw. im Bauchbereich eines Benutzers erweisen sich die hier thematisierten Probleme typischerweise im Rücken- oder Gesäßbereich als gravierender. Insofern erweist es sich als vorteilhaft, wenn der maximale Abstand der zweiten Elastifizierungsmittel voneinander (Abstand von unmittelbar nebeneinander liegenden Elastifizierungsmittel) an einem Absorptionskörperrand oder an einem Längsrand des Schrittabschnittes im Rückenabschnitt größer ist als im Bauchabschnitt.

Es wäre durchaus denkbar, dass die zweiten Elastifizierungsmittel von dem einen Seitennahtbereich zu dem anderen Seitennahtbereich durchgehend verlaufen, was insbesondere die Einbringung in einem kontinuierlichen Herstellungsverfahren im Vergleich zu einem "cut-and-place"-Prozess vereinfacht. Infolge der Überdeckung des Schrittabschnitts mit dem Bauchabschnitt und mit dem Rückenabschnitt kommt es typischerweise auch zu einer Überlappung oder Überdeckung des materialreichen Absorptionskörpers mit dem Bauchabschnitt und/oder dem Rückenabschnitt und somit auch mit demjenigen schrittseitigen und den Beinöffnungen zugewandten Bereich des Bauchabschnitts und des Rückenabschnitts, in dem die zweiten Elastifizierungsmittel verlaufen. Der materialreiche Absorptionskörper behindert dabei üblicherweise eine elastische Dehnbarkeit der Chassismaterialien. Außerdem ist es nicht unbedingt vorteilhaft, wenn der materialreiche Absorptionskörper mit zusätzlichen Spannungskräften beaufschlagt wird. Deshalb kann es sich als vorteilhaft erweisen, wenn die zweiten Elastifizierungsmittel in einem Überlappungsbereich mit dem Absorptionskörper des Schrittabschnitts hinsichtlich ihrer elastischen Eigenschaften deaktiviert sind. Diese Deaktivierung kann beispielsweise durch eine Anzahl von Trennschnitten durch die zweiten Elastifizierungsmittel im Bereich der Überdeckung mit dem Absorptionskörper realisiert werden, wobei auch andere Trennverfahren, wie z. Bsp. mittels Ultraschallschweißen oder Laser denkbar sind.

Es sei erwähnt, dass auch die ersten Elastifizierungmittel im Bereich einer Überdeckung mit dem Absorptionskörper hinsichtlich ihrer elastischen Eigenschaften deaktiviert sein können.

Es wurde bereits darauf hingewiesen, dass die zweiten Elastifizierungsmittel ungeachtet der erfindungsgemäß herbeizuführenden Spannungsverhältnisse entsprechend dem Weg ihres aufgefächerten Verlaufs bei der Herstellung des Inkontinenzartikels einer stärkeren Dehnung und damit einer höheren Vorspannung ausgesetzt sein können als in einem nicht aufgefächerten Bereich, in dem sie sich im Wesentlichen äquidistant zueinander und in der Maschinenrichtung erstrecken. Diese stärkere Vorspannung kann sich typischerweise infolge des Einbringens der zweiten Elastifizierungsmittel in einem üblichen und daher nicht im Einzelnen zu beschreibenden Stretch-Bond-Verfahren ergeben.

Hinsichtlich der Gesamtabmessungen des Inkontinenzartikels erweist es sich als vorteilhaft, wenn der Abstand (C) des schrittzugewandten innersten zweiten Elastifizierungsmittels des Bauchabschnitts von dem entsprechenden schrittzugewandten innersten zweiten Elastifizierungsmittel des Rückenabschnitts 250 bis 420 mm beträgt.

Der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts beträgt vorzugsweise 2 - 40 mm, weiter vorzugsweise 3 - 30 mm, insbesondere vorzugsweise 4 - 15 mm.

Als erste und/oder zweite Elastifizierungsmittel werden vorzugsweise faden- oder bandförmige Elastifizierungsmittel, wie Gummi- oder Polyetherpolyurethanoder Polyesterpolyurethanfäden, vorzugsweise Elastikfäden wie Lycra®- oder Spandex®-Fäden eingesetzt.

Die ersten und/oder zweiten Elastifizierungsmittel haben vorzugsweise eine Stärke von 300 - 1500 dtex, insbesondere von 500 - 900 dtex, weiter insbesondere von 500 - 600 dtex.

Die ersten und/oder zweiten Elastifizierungsmittel werden vorzugsweise unter einer Vorspannung von 1,5 - 6,0, insbesondere von 2,5 - 5,0 an den chassisbildenden Hüllmaterialien des Bauchabschnitts und Rückenabschnitts fixiert (Strech-bonding-Verfahren). Die Vorspannung ist dabei definiert als Faktor des Dehnungsgrades gegenüber dem ungedehnten/relaxierten Zustand des Elastifizierungsmittels.

Die Erstreckung des Bauchabschnitts und des Rückenabschnitts im Seitennahtbereich in Längsrichtung beträgt vorteilhafterweise wenigstens 100 mm, insbesondere wenigstens 150 mm und insbesondere 150 mm bis 220 mm.

Der minimale Abstand des Bauchabschnitts und des Rückenabschnitts in Längsrichtung voneinander beträgt vorteilhafterweise 250 bis 400 mm.

Die maximale Erstreckung des Schrittabschnitts in Querrichtung beträgt vorteilhafterweise 200 bis 350 mm, insbesondere 250 bis 320 mm.

Der Schrittabschnitt umfasst vorteilhafterweise ein flüssigkeitsundurchlässiges Backsheet-Material und ein Vlies-Topsheet-Material, zwischen denen der Absorptionskörper angeordnet ist. Hierbei bilden das Backsheet-Material und das Vlies-Topsheet-Material gewissermaßen das Chassis des Schrittabschnitts. In Weiterbildung der Erfindung von besonderer Bedeutung erweist es sich als vorteilhaft, wenn das Backsheet-Material und/oder das Topsheet-Material in Querrichtung einen Überhang über den Absorptionskörper bilden und dieser Überhang - in der Summe beidseits des Absorptionskörpers, also in der Summe links und rechts - wenigstens 25 %, insbesondere 30 - 45 % weiter insbesondere 35 - 45 %, bezogen auf die größte Breite des Schrittabschnitts (d.h. also bezogen auf die maximale Erstreckung des Schrittabschnittes in Querrichtung) beträgt.

Dieser verhältnismäßig große Überhang von Backsheet-Material und/oder Topsheet-Material beidseits der Absorptionskörpers bedeutet also einen breiten Schrittabschnitt mit einem verhältnismäßig schmalen Absorptionskörper. Hierdurch ist es möglich, entlang der Beinöffnungen erstreckte Beinelastifizierungsmittel in dem Schrittabschnitt vorzusehen, die einen verhältnismäßig großen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragkomfort des Inkontinenzartikels auswirkt.

In noch weitergehender Ausbildung der Erfindung erweist es sich als besonders vorteilhaft, wenn die Beinelastifizierungsmittel in Längsrichtung wenigstens 10 mm, insbesondere wenigstens 20 mm vor den zweiten Elastifizierungsmitteln enden. Insbesondere vorteilhaft ist, wenn die Beinelastifizierungmittel in Längsrichtung vor dem Bauchabschnitt und/oder vor dem Rückenabschnitt enden. Die von ihnen ausgeübte Spannung und Rückstellkraft beeinflusst daher nicht die erfindungsgemäß vorgesehenen Spannungsverhältnisse innerhalb des schrittseitigen und den Beinöffnungen zugewandten Bereichs des Bauchabschnitts und des Rückenabschnitts, in dem die sich auffächernden zweiten Elastifizierungsmittel vorgesehen sind.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex.

Die Beinelastifizierungsmittel werden vorzugsweise unter einer Vorspannung von 1,5 - 6,0, insbesondere von 2,5 - 4,5 an den chassisbildenden Hüllmaterialien des Schrittabschnitts fixiert.

Dessen ungeachtet erweist es sich als vorteilhaft, wenn der Bauchabschnitt und der Rückenabschnitt zumindest außerhalb des Absorptionskörpers über die Längsrichtung im Wesentlichen durchgehend flächenhaft querelastifiziert sind, wobei auch solchenfalls die erfindungsgemäßen Spannungsverhältnisse einzuhalten bzw. zu realisieren sind.

Die chassisbildenden Materialien von Bauchabschnitt und/oder Rückenabschnitt umfassen vorzugsweise Vliesstoffmaterialien, wie Spinnvliese, Kardenvliese oder Through Air bonded Kardenvliese. Besonders bevorzugt umfasst das chassisbildende Material von Bauchabschnitt und/oder Rückenabschnitt ein Spinnvliesmaterial. Die für Bauchabschnitt und/oder Rückenabschnitt eingesetzten Vliesstoffmaterialien haben vorteilhafterweise ein Flächengewicht von 10 - 30 g/m², weiter vorzugsweise von 15 - 25 g/m². Besonders bevorzugt umfassen der Bauchabschnitt und der Rückenabschnitt ein Spinnvlies aus Polypropylen, insbesondere mit einem Flächengewicht von 15 - 25 g/m².

Der Schrittabschnitt umfasst vorteilhafterweise ein flüssigkeitsundurchlässiges Backsheet-Material und ein Vlies-Topsheet-Material. Das Backsheet umfasst insbesondere eine Folie, insbesondere eines Flächengewichts von 18- 40 g/m². Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300 g/m²/24h, weiter insbesondere wenigstens 1000 g/m²/24h, weiter insbesondere wenigstens 2000 g/m²/24h, weiter insbesondere wenigstens 3000 g/m²/24h, weiter insbesondere wenigstens 4000 g/m²/24h, weiter insbesondere höchstens 6000 g/m²/24h gemessen nach DIN 53 122-1 (Ausgabe: 2001-08).

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskörper außerdem superabsorbierende Materialien (SAP), insbesondere auf Basis oberflächenvernetzter, teilneutralisierter Polyacrylate.

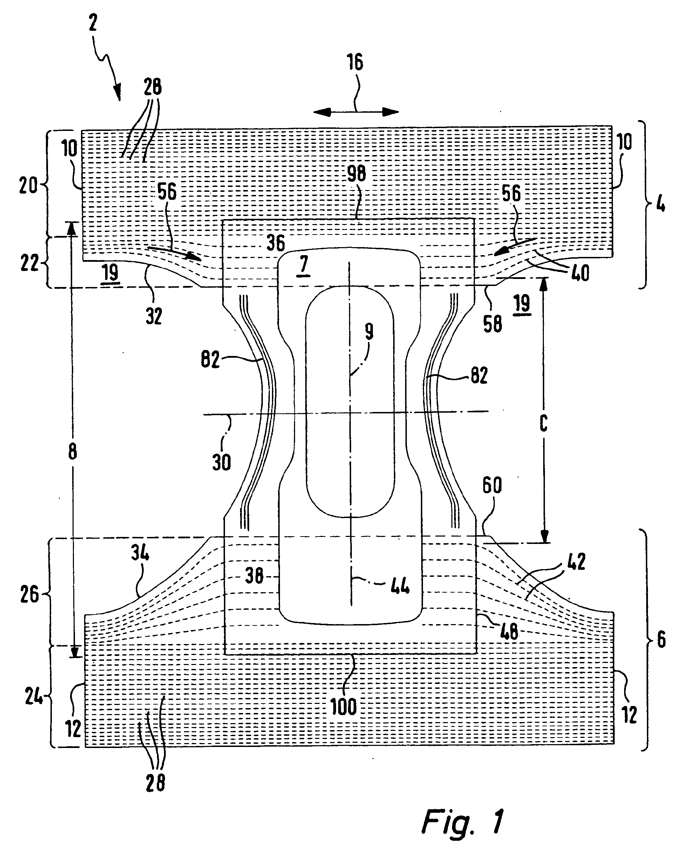

Es erweist sich weiter als vorteilhaft, wenn der Anteil der Fläche des Schrittabschnitts an der gesamten Fläche des Inkontinenzartikels 25 - 55 % beträgt, und wenn der Schrittabschnitt wenigstens 12 % der Fläche des Bauchabschnitts und wenigstens 20 % der Fläche des Rückenabschnitts überlappt. Die Flächenanteile werden jeweils im ausgedehnten Zustand der Chassismaterialien wie in Figur 1 dargestellt bestimmt.

Es erweist sich als vorteilhaft, wenn der Schrittabschnitt 15 - 40 %, insbesondere 15 - 35 % und weiter insbesondere 15 - 25 % der Fläche des Bauchabschnitts überlappt.

Ferner erweist es sich als vorteilhaft, wenn der Schrittabschnitt den Bauchabschnitt mit einer Fläche von 25.000 - 45.000 mm² überlappt.

Ferner erweist es sich als vorteilhaft, wenn der Schrittabschnitt 20 - 40 %, insbesondere 20 - 35 % und weiter insbesondere 22 - 32 % der Fläche des Rückenabschnitts überlappt.

Ferner erweist es sich als vorteilhaft, wenn der Schrittabschnitt den Rückenabschnitt mit einer Fläche von 35.000 - 65.000 mm², insbesondere von 40.000 - 55.000 mm² überlappt.

Ferner erweist es sich als vorteilhaft, wenn die Überlappung des Schrittabschnitts mit dem Rückenabschnitt größer ist als mit dem Bauchabschnitt.

Der Schrittabschnitt bzw. die Längsränder des Schrittabschnitts, welche die Beinöffnungen begrenzen, sind vorteilhafterweise bogenförmig konturiert ausgebildet.

Ferner erweist es sich als vorteilhaft, wenn auch der Absorptionskörper 5 - 20 %, insbesondere 5 - 15 % der Fläche des Bauchabschnitts und/oder 10 - 20 %, insbesondere 10 - 15 % der Fläche des Rückenabschnitts überlappt.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1          eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figur 2    eine ausschnittsweise Darstellung des Inkontinenzartikels nach Figur 1;

Figur 3    eine Draufsicht auf den Schrittabschnitt des Inkontinenzartikels nach Figur 1 wie-derum im flachgelegten und ausgedehn-ten Zustand;

Figur 4    eine Schnittansicht (schematisch) ent-lang einer Quermittelachse des Schritt-abschnitts mit Schnittebene IV-IV in Figur 3;

Figur 5    eine Figur 4 entsprechende Schnittansicht (schematisch) des Schrittabschnitts mit Schnittebene V-V in Figur 3, mit ent-falteten und emporstehenden Barriere-mitteln;

Figur 6    eine perspektivische Ansicht (schematisch) des an einen Benutzer angelegten Inkontinenzartikels nach Figur 1;

Figur 7    eine Figur 1 entsprechende Draufsicht des erfindungsgemäßen Inkontinenzartikels zur Verdeutlichung der Verbindung von Schrittabschnitt und Bauchabschnitt bzw. Rückenabschnitt und

Figuren 8,9    verdeutlichen beispielhaft die Bestim-mung von Rückstellkräften im Bauchab-schnitt bzw. Rückkenabschnitt des erfin-dungsgemäßen Inkontinenzartikels.

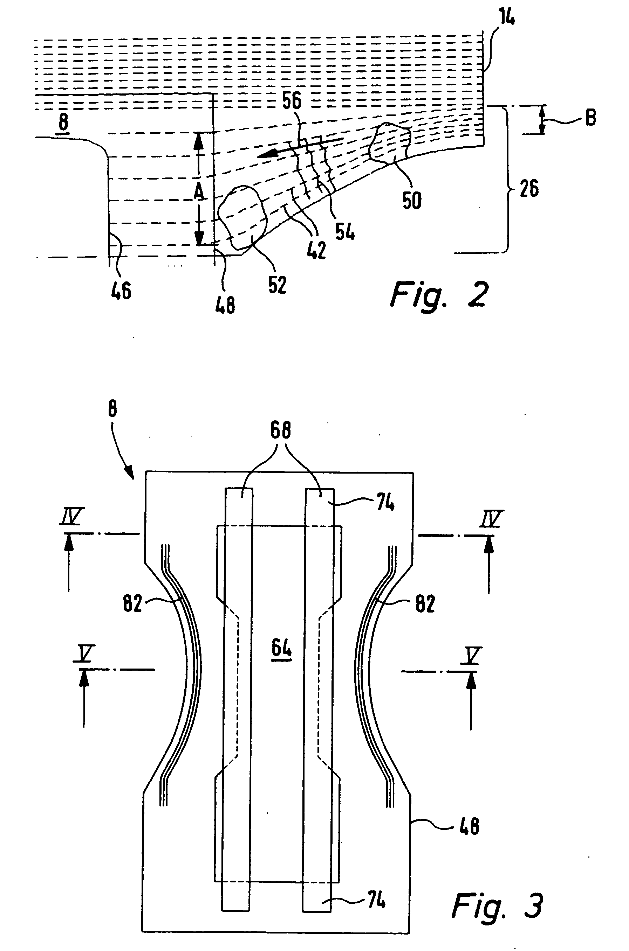

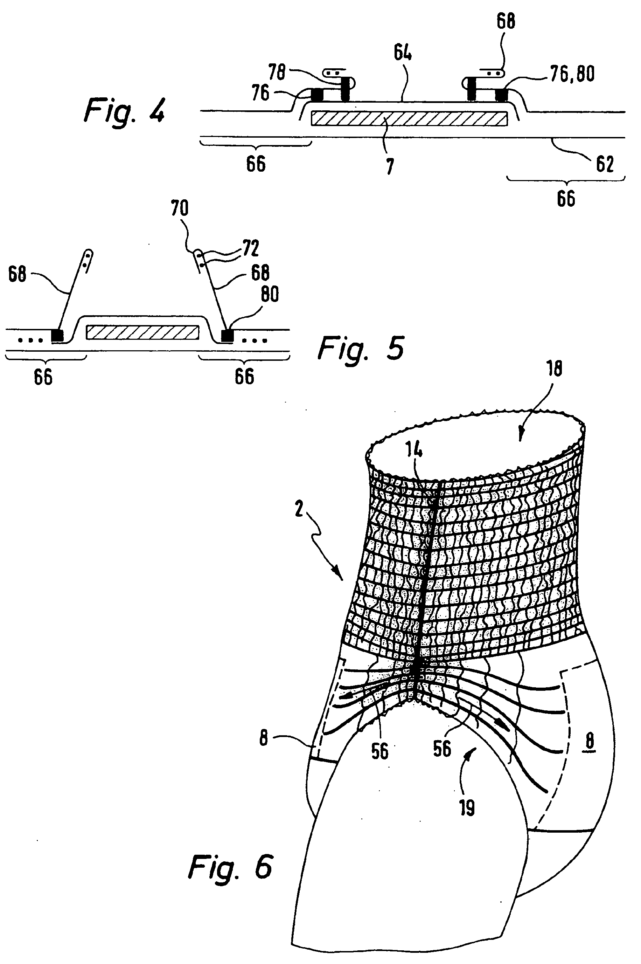

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil des Bauchabschnitts 4 einerseits und des Rückenabschnitts 6 andererseits überlappt und im Überlappungsbereich herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der in Figur 6 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 6) ausgebildet werden. In die-sem herstellerseitig gefertigten höschenförmigen Zu-stand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.
Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.
In dem hüftseitigen Bereich 20 des Bauchabschnitts 4 und in dem hüftseitigen Bereich 24 des Rük-kenabschnitts 6 sind erste Elastifizierungsmittel 28 vorgesehen, bei denen es sich insbesondere um fadenförmige Elastifizierungsmittel, wie Lycra-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.
Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist. Je größer der Überlappungsbereich 36, 38, umso geringere Klebermengen bezogen auf Fläche des Überlappungsbereiches können verwendet werden, was sich im Hinblick auf die Steifigkeit der Chassismaterialien als vorteilhaft auswirkt. Insbesondere kann ein nicht vollflächiger Kleberauftrag zur Verbindung der Komponenten verwendet werden.
Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 2 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 2 auch quantitativ näher bezeichnen. Beispielsweise haben die in Figur 2 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 2 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden Elastifizierungsmitteln), und zwar A als der maximale Abstand, insbesondere am Längsrand 48 des Schrittabschnitts 8, und B als der minimale Abstand, insbesondere im Seitennahtbereich 14.

Die Ausbildung des schrittseitigen und den Beinöffnungen 19 zugewandten Bereichs 22 bzw. 26 mit den zweiten Elastifizierungsmitteln 40, 42 und die Ausbildung des in Längsrichtung 9 daran anschließenden hüftseitigen Bereichs 20 bzw. 24 mit den ersten Elastifizierungsmitteln 28 ist erfindungsgemäß derart, dass die bei flächenhafter Dehnung auftretende Rückstellkraft in dem schrittseitigen und den Beinöffnungen 19 zugewandten Bereich 22 bzw. 26 geringer ist als in dem in Längsrichtung 9 daran anschließenden hüftseitigen Bereich 20, 24. Durch die erfindungsgemäß bewusst vorgesehene Verringerung dieser Rückstellkraft in dem schrittzugewandten Bereich 22, 26 gegenüber dem in Längsrichtung 9 daran anschließenden hüftseitigen Bereich 20, 24 wird ein zuvor nicht realisierter Freiheitsgrad geschaffen, mit dem die eingangs geschilderten Probleme besser überwunden werden.

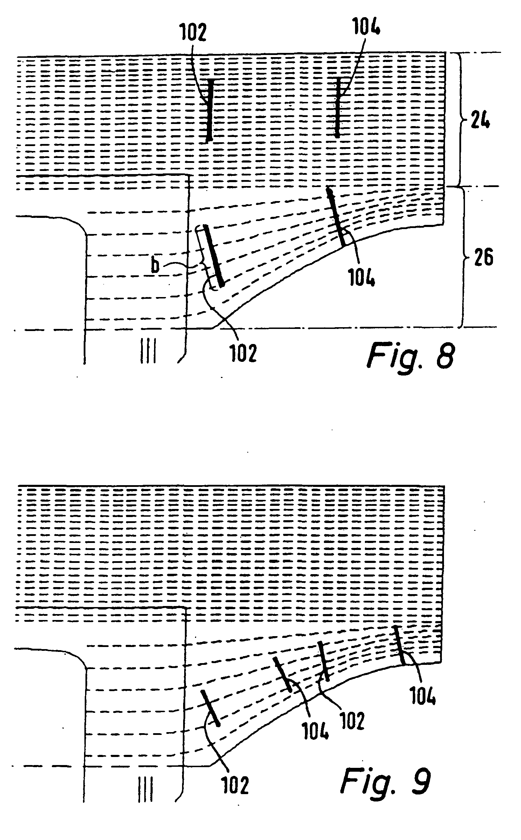

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts 6 zwischen zwei Klemmbacken 102, 104 (siehe Figur 8) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstands im ungespannten Zustand) ermittelt. Die Klemmbacken 102, 104 werden dabei jeweils voneinander wegbewegt. Die Klemmbacken 102, 104 sollten möglichst viele, zumindest jedoch zwei nebeneinander angeordnete Elastifizierungsmittel 40, 42 bzw. 28 des zu messenden Bereichs fixieren, und sie sollten im Wesentlichen rechtwinklig zum Verlauf der Elastifizierungsmittel orientiert sein, so dass die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figur 8 verdeutlicht.

Sofern der Auffächerungsgrad bei den zweiten Elastifizierungsmitteln 40, 42 hinreichend groß gewählt wird, so lässt sich auf diese Weise zusätzlich eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt, geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung 56 auf den Schrittabschnitt 8. Dadurch, dass die sich bei flächenhafter Dehnung in dem schrittseitigen und den Beinöffnungen zugewandten Bereich 22 des Bauchabschnitts 4 beziehungsweise 26 des Rückenabschnitts 6 einstellenden Rückstellkräfte in Richtung des Pfeils 56, also generell vom Seitennahtbereich 14 in Richtung auf den Schrittabschnitt 8 abnehmen, wird eine erhebliche weitere Verbesserung des Tragekomforts erzielt, weil gerade in diesen Bereichen - wie erfindungsgemäß festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Figur 9 zeigt prinzipiell die Anordnung von Klemmbacken 102, 104, um die Rückstellkräfte in einem Gebiet näher am Schrittabschnitt 8 mit den Rückstellkräften in einem Gebiet näher am Seitennahtbereich 14 zu vergleichen.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 in Längsrichtung 9 voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 - 15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die maximale Erstreckung des Schrittabschnitts 8 in Querrichtung 16 beträgt vorteilhafterweise 200 bis 350 mm.

Der Schrittabschnitt 8 umfasst ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist wie aus den Figuren 4, 5 ersichtlich der Absorptionskörper 7 angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 5 schematisch dargestellt ist. An ihren jeweiligen Längsendbereichen 74 sind die seitlichen Barrieremittel 68 über schematisch angedeutete Fixierungen 76, 78 auf das Topsheet 64 beziehungsweise auf sich selbst in einer C-förmig gefalteten Konfiguration festgelegt. Vorteilhaft und erwähnenswert ist hier, dass die in der Figur 4 jeweils innenliegende Fixierung 78 das Barrieremittel 68 auf sich selbst festlegt, und zwar in Querrichtung 16 innerhalb der äußeren Fixierung 76, welche eine in Längsrichtung 9 durchgehend erstreckte Cuffsockellinie 80 bildet. Die innere Fixierung 78 ist hingegen nur in den Längsendbereichen 74 der Barrieremittel 68 vorgesehen.

Es erweist sich hier als besonders vorteilhaft, wenn der erwähnte Überhang 66 des Backsheet-Materials 62 und/oder des Topsheet-Materials 64 über den Absorptionskörper 7 - in der Summe beidseits des Absorptionskörpers, also in der Summe links und rechts - wenigstens 25 % bezogen auf die größte Breite des Schrittabschnitts 8 beträgt. Auf diese Weise ist nämlich in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das erfindungsgemäß zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 1 ersichtlich ist, ist bei dem Schrittabschnitt 8 ein in Querrichtung verhältnismäßig großer Überhang 66 über den Absorptionskörper 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schrittabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann, und zwar auch, wenn kein vollflächiger Kleberauftrag verwendet wird. Es ist solchenfalls in vorteilhafter Weise hinreichend, wenn ein nur abschnittsweiser oder gerasterter Kleberauftrag zur Verbindung verwendet wird. Dies ist deshalb vorteilhaft, weil solchenfalls die miteinander gefügten Materialien nicht zu steif werden.

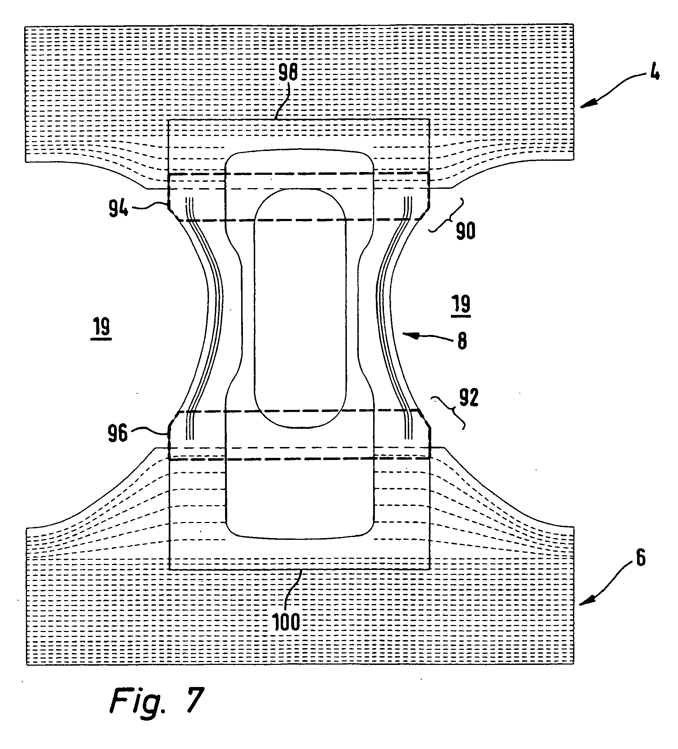

Anhand der Figur 7, die der Figur 1 entspricht, wird ein weiteres vorteilhaftes Detail des erfindungsgemäßen Inkontinenzartikels erläutert. Durch die Verfolgung des dreikomponentigen Konzepts zur Herstellung des erfindungsgemäßen Inkontinenzartikels ergibt sich ein Übergang 90 zwischen Schrittabschnitt 8 und Bauchabschnitt 4 sowie ein Übergang 92 zwischen Schrittabschnitt 8 und Rückenabschnitt 6, bei denen sich üblicherweise ein unstetiger, also mit Ecken oder Winkeln oder Knicken versehener Verlauf der die Beinöffnungen 19 begrenzenden Ränder der Chassismaterialien ergibt. Dies birgt die Gefahr, dass im Bereich der Übergänge 90, 92 Kraftspitzen gebildet werden, die zu einem Einreißen der Chassismaterialien führen können, was die Anfügung des Schrittabschnitts 8 an den Bauchabschnitt 4 beziehungsweise an den Rückenabschnitt 6 beeinträchtigen kann. Um dem entgegen zu wirken ist im jeweiligen Übergang 90 und 92 eine verstärkende Beschichtung 94, 96 des flüssigkeitsundurchlässigen Backsheet-Materials 62 des Schrittabschnitts 8 vorgesehen. Es erweist sich als hinreichend, wenn diese verstärkende Beschichtung 94, 96 nur in dem durch die unterbrochene Linie jeweils bezeichneten Bereich der Figur 7 vorgesehen wird. Die verstärkende Beschichtung 94, 96 überlappt im beispielhaft und vorzugsweise dargestellten Fall den Bauchabschnitt 4 und den Rückenabschnitt 6 in Längsrichtung 9 lediglich um etwa 10 bis 20 mm, insbesondere um ca. 15 mm. Die verstärkende Beschichtung endet in Längsrichtung 9 jeweils vor den Längsenden 98, 100 des Schrittabschnitts, und zwar wenigstens 30 mm vor dem bauchseitigen Längsende 98 und wenigstens 90 mm vor dem rückenseitigen Längsende 100. Dies erweist sich als vorteilhaft, da solchenfalls die verstärkende Beschichtung 94, 96 nicht unnötig zur Versteifung der Chassismaterialien in Bereichen beiträgt, wo dies nicht hilfreich, sondern eher unerwünscht und nachteilig ist. Außerdem können auf diese Weise Materialkosten gespart werden. Jedoch bleibt die Möglichkeit unberührt, die verstärkende Beschichtung 94, 96 nicht nur im Übergang 90 beziehungsweise 92 vorzusehen.

Die verstärkende Beschichtung 94, 96 besteht vorzugsweise aus einem Vliesstoff, insbesondere aus einem Spinnvlies aus Polypropylen, insbesondere mit einem Flächengewicht von 10 - 20 g/m$^2$ , insbesondere von 12 - 17 g/m$^2$.

## Patentansprüche

1.  Inkontinenzartikel (2) in Höschenform für die Aufnahme von Körperausscheidungen mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in einer Längsrichtung (9) zwischen Bauchabschnitt (4) und Rückenabschnitt (6) erstreckt und an den Bauchabschnitt (4) und an den Rückenabschnitt (6) unlösbar angefügt ist, wobei sowohl der Schrittabschnitt (8) als auch der Bauchabschnitt (4) und der Rückenabschnitt (6) Beinöffnungen (19) des Inkontinenzartikels begrenzen und der Bauchabschnitt (4) und der Rückenabschnitt (6) hierfür eine von der Quer- oder Hüftumfangsrichtung (16) abweichende und in Richtung auf eine Quermittelachse (30) des Schrittabschnitts (8) zulaufende Randkontur (32, 34) zur Begrenzung der Beinöffnungen (19) aufweisen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, die sich ausgehend von den beiden Seitennahtbereichen (14) in Richtung auf eine Längsmittelachse (44) des Inkontinenzartikels erstrecken und dabei bogenförmig sich auffächernd mit zunehmendem Abstand voneinander verlaufen, und wobei in diesem schrittseitigen und den Beinöffnungen zugewandten Bereich (22, 26) des Bauchabschnitts (4) und des Rückenabschnitts (6) mit auffächerndem Verlauf der zweiten Elastifizierungsmittel (40, 42) die bei flächenhafter Dehnung dieses Bereichs (22, 26) auftretende Rückstellkraft geringer ist als bei flächenhafter Dehnung in einem hüftseitigen Bereich (20, 24), in welchem nur die ersten Elastifizierungsmittel (28) vorgesehen sind.

2.  Inkontinenzartikel nach Anspruch 1, **dadurch gekennzeichnet, dass** die bei flächenhafter Dehnung dieses schrittseitigen und den Beinöffnungen zugewandten Bereichs (22, 26) auftretende Rückstellkraft zusätzlich innerhalb dieses Bereichs (22, 26) in Richtung (56) auf den Schrittabschnitt (8) und eine Längsmittelachse (44) des Inkontinenzartikels abnimmt.

3. Inkontinenzartikel nach Anspruch 1 oder 2, **dadurch gekennzeichnet, dass** ein minimaler Abstand der zweiten Elastifizierungsmittel (40, 42) voneinander in den Seitennahtbereichen (14) 3 - 8 mm, insbesondere 3 - 7 mm und weiter insbesondere 3 - 6 mm beträgt.

4. Inkontinenzartikel nach einem oder mehreren der vorstehenden Ansprüche, **dadurch gekennzeichnet, dass** ein maximaler Abstand der zweiten Elastifizierungsmittel (40, 42) voneinander an einem Absorptionskörperrand (46) oder an einem Längsrand (48) des Schrittabschnitts (8) 7 - 35 mm, insbesondere 10 - 32 mm und weiter insbesondere 12 - 30 mm beträgt.

5. Inkontinenzartikel nach einem oder mehreren der vorstehenden Ansprüche, **dadurch gekennzeichnet, dass** die zweiten Elastifizierungsmittel (40, 42) einen Auffächerungsgrad (F=(A-B)/B * 100%) von 50 - 900 %, insbesondere von 100 - 700 % und weiter insbesondere von 150 - 550 % haben.

6. Inkontinenzartikel nach Anspruch 6, **dadurch gekennzeichnet, dass** der Auffächerungsgrad (F= (AB)/B * 100%) der zweiten Elastifizierungsmittel (40, 42) im Rückenabschnitt (6) größer ist als im Bauchabschnitt (4).

7. Inkontinenzartikel nach einem oder mehreren der vorstehenden Ansprüche, **dadurch gekennzeichnet, dass** der maximale Abstand der zweiten Elastifizierungsmittel (40, 42) voneinander an einem Absorptionskörperrand (46) oder an einem Längsrand (48) des Schrittabschnitts (8) im Rückenabschnitt (6) größer ist als im Bauchabschnitt (4).

8. Inkontinenzartikel nach einem oder mehreren der vorstehenden Ansprüche, **dadurch gekennzeichnet, dass** die zweiten Elastifizierungsmittel (40, 42) in einem Überlappungsbereich (36, 38) mit dem Absorptionskörper (7) des Schrittabschnitts (8) hinsichtlich ihrer elastischen Eigenschaften deaktiviert sind.

9. Inkontinenzartikel nach einem oder mehreren der vorstehenden Ansprüche, **dadurch gekennzeichnet, dass** die zweiten Elastifizierungsmittel (40, 42) entsprechend dem Weg ihres aufgefächerten Verlaufs stärker vorgespannt sind als in einem nicht aufgefächerten Bereich.

10. Inkontinenzartikel nach einem oder mehreren der vorstehenden Ansprüche, **dadurch gekennzeichnet, dass** der Abstand (C) des schrittzugewandten innersten zweiten Elastifizierungsmittels (40, 42) des Bauchabschnitts (4) von dem entsprechenden schrittzugewandten innersten zweiten Elastifizierungsmittel (40, 42) des Rückenabschnitts (6) 250 - 420 mm beträgt.

11. Inkontinenzartikel nach einem oder mehreren der vorstehenden Ansprüche, **dadurch gekennzeichnet, dass** der Abstand der innersten, schrittzugewandten zweiten Elastifizierungsmittel (40, 42) von der die Beinöffnungen begrenzenden Randkontur (32, 34) des schrittseitigen und den Beinöffnungen zugewandten Bereichs (22, 26) des Bauchabschnitts (4) und des Rückenabschnitts (6) 2 - 40 mm, vorzugsweise 3 - 30 mm, insbesondere vorzugsweise 4 - 15 mm beträgt.

12. Inkontinenzartikel nach einem oder mehreren der vorstehenden Ansprüche, **dadurch gekennzeichnet, dass** die Erstreckung des Bauchabschnitts (4) und des Rückenabschnitts (6) im Seitennahtbereich (14) in Längsrichtung (9) wenigstens 100 mm, insbesondere wenigstens 150 mm und insbesondere 150 mm - 220 mm beträgt.

13. Inkontinenzartikel nach einem oder mehreren der vorstehenden Ansprüche, **dadurch gekennzeichnet, dass** der minimale Abstand des Bauchabschnitts (4) und des Rückenabschnitts (6) in Längsrichtung (9) voneinander 250 - 400 mm beträgt.

14. Inkontinenzartikel nach einem oder mehreren der vorstehenden Ansprüche, **dadurch gekennzeichnet, dass** die maximale Erstreckung des Schrittabschnitts (8) in Querrichtung (16) 200 - 350 mm, insbesondere 250-320 mm beträgt.

15. Inkontinenzartikel nach einem oder mehreren der vorstehenden Ansprüche, **dadurch gekennzeichnet, dass** der Schrittabschnitt (8) ein flüssigkeitsundurchlässiges Backsheet-Material (62) und ein Topsheet-Material (64) umfasst, zwischen denen der Absorptionskörper (7) angeordnet ist, und das Backsheet-Material (62) und/oder das Topsheet-Material (64) in Querrichtung (16) einen Überhang (66) über den Absorptionskörper (7) bilden und dieser Überhang (66) - in der Summe beidseits des Absorptionskörpers, also in der Summe links und rechts - wenigstens 25 %, insbesondere 30 - 45 % und weiter insbesondere 35 - 45 % bezogen auf die größte Breite des Schrittabschnitts (8) beträgt.

16. Inkontinenzartikel nach einem oder mehreren der vorstehenden Ansprüche, **dadurch gekennzeichnet, dass** der Schrittabschnitt (8) außerhalb des Absorptionskörpers (7) entlang der Beinöffnungen (19) erstreckte Beinelastifizierungsmittel (82) aufweist, die in Längsrichtung (9) wenigstens 10 mm, insbesondere wenigstens 20 mm vor den zweiten Elastifizierungsmitteln (40, 42) enden.

17. Inkontinenzartikel nach einem oder mehreren der vorstehenden Ansprüche, **dadurch gekennzeichnet, dass** die Beinelastifizierungsmittel (82) in Längsrichtung (9) vor dem Bauchabschnitt (4) und/oder vor dem Rükkenabschnitt (6) enden.

18. Inkontinenzartikel nach einem oder mehreren der vorstehenden Ansprüche, **dadurch gekennzeichnet, dass** der Bauchabschnitt (4) und der Rückenabschnitt (6) zumindest außerhalb des Absorptionskörpers (7) über die Längsrichtung (9) im wesentlichen durchgehend flächenhaft querelastifiziert sind.

19. Inkontinenzartikel nach einem oder mehreren der vorstehenden Ansprüche, **dadurch gekennzeichnet, dass** der Anteil der Fläche des Schrittabschnitts (8) an der gesamten Fläche des Inkontinenzartikels 25 - 55 % beträgt, und dass der Schrittabschnitt (8) wenigstens 12 % der Fläche des Bauchabschnitts (4) und wenigstens 20 % der Fläche des Rückenabschnitts (6) überlappt (Flächenanteile bestimmt jeweils im ausgedehnten Zustand der Chassismaterialien wie in Figur 1 dargestellt).

20. Inkontinenzartikel nach einem oder mehreren der vorstehenden Ansprüche, **dadurch gekennzeichnet, dass** der Schrittabschnitt (8) 15 - 40 %, insbesondere 15 - 35 % und weiter insbesondere 15 - 25 % der Fläche des Bauchabschnitts (4) überlappt und/oder dass der Schrittabschnitt (8) den Bauchabschnitt (4) mit einer Fläche von 25.000 - 45.000 mm² überlappt.

21. Inkontinenzartikel nach einem oder mehreren der vorstehenden Ansprüche, **dadurch gekennzeichnet, dass** der Schrittabschnitt (8) 20 - 40 %, insbesondere 20 - 35 % und weiter insbesondere 22 - 32 % der Fläche des Rückenabschnitts (6) überlappt und/oder dass der Schrittabschnitt (8) den Rückenabschnitt (6) mit einer Fläche von 35.000 - 65.000 mm², insbesondere von 40.000 - 55.000 mm² überlappt.

22. Inkontinenzartikel nach einem oder mehreren der vorstehenden Ansprüche, **dadurch gekennzeichnet, dass** die Überlappung des Schrittabschnitts (8) mit dem Rückenabschnitt (6) größer ist als mit dem Bauchabschnitt (4).

23. Inkontinenzartikel nach einem oder mehreren der vorstehenden Ansprüche, **dadurch gekennzeichnet, dass** auch der Absorptionskörper (7) 5 - 20 %, insbesondere 5 - 15 % der Fläche des Bauchabschnitts (4) und/oder 10 - 20 %, insbesondere 10 - 15 % der Fläche des Rückenabschnitts (6) überlappt.

## Claims

1. Incontinence article (2) in the form of pants for receiving body excrements, comprising a front belly section (4) and a rear back section (6), which are connected to each other at side seam areas (14) on both sides during production to form a belly and back band which is continuous in the transverse or peripheral hip direction and has a hip opening (18) which is closed in the peripheral hip direction, and a crotch section (8) which comprises an absorbent body (7) and extends in a longitudinal direction (9) between the belly section (4) and the back section (6) and is permanently attached to the belly section (4) and the back section (6), wherein the crotch section (8) as well as the belly section (4) and the back section (6) define leg openings (19) of the incontinence article, and the belly section (4) and the back section (6) have an edge contour (32, 34) for defining the leg openings (19), which extends towards a transverse center axis (30) of the crotch section (8) and differs from the transverse or peripheral hip direction (16), wherein first elastification means (28) are provided in the belly section (4) and the back section (6), which extend at a separation from each other and parallel to each other in the transverse or peripheral hip direction (16) and thereby extensively elasticise the belly section (4) and the back section (6), wherein, in an area (22, 26) of the belly section (4) and the back section (6) on the crotch side facing the leg openings (19), second elastification means (40, 42) are provided, which extend starting from the two side seam areas (14) towards a longitudinal center axis (44) of the incontinence article in a curved shape, thereby fanning out with increasing separation from each other, and wherein, in this area (22, 26) of the belly section (4) and the back section (6) on the crotch side facing the leg openings, where the second elastification means (40, 42) fan out, the restoring force generated through extensive stretching of this area (22, 26) is smaller than that generated through extensive stretching of an area (20, 24) on the hip side where only the first elastification means (28) are provided.

2. Incontinence article according to claim 1, **characterized in that** the restoring force generated through extensive stretching of the area (22, 26) on the crotch side facing the leg openings additionally decreases within this area (22, 26) in the direction (56) towards the crotch section (8) and a longitudinal center axis (44) of the incontinence article.

3. Incontinence article according to claim 1 or 2, **characterized in that** a minimum separation between the second elastification means (40, 42) in the side seam areas (14) is 3 to 8 mm, in particular 3 to 7 mm, and moreover, in particular 3 to 6 mm.

4. Incontinence article according to any one or more of the preceding claims, **characterized in that** a maximum separation between the second elastification means (40, 42) at an absorbent body edge (46) at on a longitudinal edge (48) of the crotch section (8) is 7 to 35 mm, in particular 10 to 32 mm, and moreover, in particular 12 to 30 mm.

5. Incontinence article according to any one or more of the preceding claims, **characterized in that** the second elastification means (40, 42) have a fanning-out degree (F=(A-B)/B * 100%) of 50 to 900 %, in particular 100 to 700 % and moreover, in particular 150 to 550 %.

6. Incontinence article according to claim 5, **characterized in that** the fanning-out degree (F=(A-B)/B * 100%) of the second elastification means (40, 42) in the back section (6) is larger than in the belly section (4).

7. Incontinence article according to any one or more of the preceding claims, **characterized in that** the maximum separation between the second elastification means (40, 42) at an absorbent body edge (46) or at a longitudinal edge (48) of the crotch section (8) in the back section (6) is larger than in the belly section (4).

8. Incontinence article according to any one or more of the preceding claims, **characterized in that** the elastic properties of the second elastification means (40, 42) are deactivated in an overlapping area (36, 38) with the absorbent body (7) of the crotch section (8).

9. Incontinence article according to any one or more of the preceding claims, **characterized in that** the second elastification means (40, 42) have a greater pretension in correspondence with their fanned-out shape than in an area where they are not fanned out.

10. Incontinence article according to any one or more of the preceding claims, **characterized in that** the separation (C) between the innermost second elastification means (40, 42) of the belly section (4) facing the crotch area, and the corresponding innermost second elastification means (40, 42) of the back section (6) facing the crotch area is 250 to 420 mm.

11. Incontinence article according to any one or more of the preceding claims, **characterized in that** the separation between the innermost second elastification means (40, 42) facing the crotch area and the edge contour (32, 34), delimiting the leg openings, of the area (22, 26) of the belly section (4) and the back section (6) on the crotch side facing the leg openings, is 2 to 40 mm, preferably 3 to 30 mm, in particular preferably 4 to 15 mm.

12. Incontinence article according to any one or more of the preceding claims, **characterized in that** the belly section (4) and the back section (6) extend in the side seam area (14) in the longitudinal direction (9) by at least 100 mm, in particular at least 150 mm, and, in particular 150 mm to 220 mm.

13. Incontinence article according to any one or more of the preceding claims, **characterized in that** the minimum separation between the belly section (4) and the back section (6) in the longitudinal direction (9) is 250 to 400 mm.

14. Incontinence article according to any one or more of the preceding claims, **characterized in that** the maximum extension of the crotch section (8) in the transverse direction (16) is 200 to 350 mm, in particular 250 to 320 mm.

15. Incontinence article according to any one or more of the preceding claims, **characterized in that** the crotch section (8) comprises a liquid-impermeable backsheet material (62) and a topsheet material (64) between which the absorbent body (7) is disposed, and the backsheet material (62) and/or the topsheet material (64) form a projection (66) that extends past the absorbent body (7) in the transverse direction (16), and the projection (66) on both sides of the absorbent body, i.e. on the left-hand and right-hand side added together, is at least 25 %, in particular 30 to 45 %, and moreover, in particular 35 to 45 %, with respect to the maximum width of the crotch section (8).

16. Incontinence article according to any one or more of the preceding claims, **characterized in that** the crotch section (8) has leg elastification means (82) which extend outside of the absorbent body (7) along the leg openings (19) and terminate in the longitudinal direction (9) at least 10 mm, in particular at least 20 mm upstream of the second elastification means (40, 42).

17. Incontinence article according to any one or more of the preceding claims, **characterized in that** the leg elastification means (82) terminate in the longitudinal direction (9) upstream of the belly section (4) and/or upstream of the back section (6).

18. Incontinence article according to any one or more of the preceding claims, **characterized in that** the belly section (4) and the back section (6) are substantially continuously extensively transversely elasticised across the longitudinal direction (9) at least outside of the absorbent body (7).

19. Incontinence article according to any one or more of the preceding claims, **characterized in that** the proportion of the surface of the crotch section (8) with respect to the entire surface of the incontinence article is 25 to 55 %, and the crotch section (8) overlaps at least 12 % of the surface of the belly section (4) and at least 20 % of the surface of the back section (6) (surface portions determined each time in the extended state of the chassis materials as shown in Fig. 1).

20. Incontinence article according to any one or more of the preceding claims, **characterized in that** the crotch section (8) overlaps 15 to 40 %, in particular 15 to 35 %, and moreover, in particular 15 to 25 % of the surface of the belly section (4) and/or that the crotch section (8) overlaps the belly section (4) with a surface of 25,000 to 45,000 mm$^2$.

21. Incontinence article according to any one or more of the preceding claims, **characterized in that** the crotch section (8) overlaps 20 to 40 %, in particular 20 to 35 %, and moreover, in particular 22 to 32 % of the surface of the back section (6) and/or that the crotch section (8) overlaps the back section (6) with a surface of 35,000 to 65,000 mm$^2$, in particular, 40,000 to 55,000 mm$^2$.

22. Incontinence article according to any one or more of the preceding claims, **characterized in that** the overlap between the crotch section (8) and the back section (6) is larger than with the belly section (4).

23. Incontinence article according to any one or more of the preceding claims, **characterized in that** the absorbent body (7) also overlaps 5 to 20 %, in particular 5 to 15 %, of the surface of the belly section (4) and/or 10 to 20 %, in particular 10 to 15 %, of the surface of the back section (6).

**Revendications**

1. Article pour personnes incontinentes (2) se présentant sous la forme d'une culotte, destiné à recevoir les excrétions corporelles, comprenant une section ventrale avant (4) et une section dorsale arrière (6), lesquelles sont reliées entre elles en usine au niveau des deux côtés des zones de couture latérales (14) pour former une bande ventrale et dorsale d'un seul tenant dans le sens transversal ou dans le sens de la circonférence des hanches (16) avec une ouverture de hanche (18) fermée dans le sens de la circonférence des hanches, et comprenant une section d'entrejambe (8) présentant un corps absorbant (7), laquelle section d'entrejambe s'étend dans le sens longitudinal (9) entre la section ventrale (4) et la section dorsale (6) et est jointe de façon inamovible à la section ventrale (4) et à la section dorsale (6), dans lequel à la fois la section d'entrejambe (8) et la section ventrale (4) et la section dorsale (6) définissent des ouvertures de jambes (19) de l'article pour personnes incontinentes, la section ventrale (4) et la section dorsale (6) présentant à cet effet un contour de bord (32, 34) qui s'étend en s'écartant de la direction transversale ou de la circonférence de hanche (16) en direction d'un axe médian transversal (30) de la section d'entrejambe (8) afin de délimiter les ouvertures de jambes (19), dans lequel il est prévu dans la section ventrale (4) et dans la section dorsale (6) des premiers moyens d'élastification (28) qui s'étendent à distance les uns des autres et parallèlement entre eux en direction transversale ou dans le sens de la circonférence de hanche (16) et élastifient ainsi de façon plane la section ventrale (4) et la section dorsale (6), des deuxièmes moyens d'élastification (40, 42) étant prévus dans une zone (22, 26) de la section ventrale (4) et de la section dorsale (6) orientée vers l'entrejambe et les ouvertures de jambes (19), lesquels deuxièmes moyens d'élastification s'étendent depuis les deux zones de couture latérales (14) en direction d'un axe médian longitudinal (44) de l'article pour personnes incontinentes et s'étendent de façon incurvée de plus en plus distancés les uns des autres, et dans lequel dans cette zone (22, 26) de la section ventrale (4) et de la section dorsale (6) orientée vers l'entrejambe et les ouvertures de jambes avec un tracé en éventail des deuxièmes moyens d'élastification (40, 42), la force de rappel qui apparaît lors de l'allongement de cette zone (22, 26) est inférieure à celle qui apparaît lors de l'allongement dans une zone côté hanche (20, 24) dans laquelle il n'est prévu que les premiers moyens d'élastification (28).

2. Article pour personnes incontinentes selon la revendication 1, **caractérisé en ce que** la force de rappel qui apparaît lors de l'allongement de cette zone (22, 26) orientée vers l'entrejambe et les ouvertures de jambes augmente en outre à l'intérieur de cette zone (22, 26) en direction (56) de la section d'entrejambe (8) et d'un axe médian longitudinal (44) de l'article pour personnes incontinentes.

3. Article pour personnes incontinentes selon la revendication 2, **caractérisé en ce que** la distance minimale entre les deuxièmes moyens d'élastification (40, 42) dans les zones de couture latérales (14) est comprise entre 3 et 8 mm, en particulier entre 3 et 7 mm et plus particulièrement entre 3 et 6 mm.

4. Article pour personnes incontinentes selon une ou plusieurs des revendications précédentes, **caractérisé en ce que** la distance maximale entre les deuxièmes moyens d'élastification (40, 42) au niveau du bord du corps absorbant (46) ou bien au niveau d'un bord longitudinal (48) de la section d'entrejambe (8) est comprise entre 7 et 35 mm, en particulier entre 10 et 32 mm et plus particulièrement entre 12 et 30 mm.

5. Article pour personnes incontinentes selon une ou plusieurs des revendications précédentes, **caractérisé en ce que** les deuxièmes moyens d'élastification (40, 42) ont un degré d'élargissement en éventail (F = (A-B)/B * 100 %) allant de 50 à 900 %, en particulier de 100 à 700 % et plus particulièrement de 150 à 550 %.

6. Article pour personnes incontinentes selon la revendication 5, **caractérisé en ce que** le degré d'élargissement en éventail (F = (A-B)/B * 100 %) des deuxièmes moyens d'élastification (40, 42) est plus grand dans la section dorsale (6) que dans la section ventrale (4).

7. Article pour personnes incontinentes selon une ou plusieurs des revendications précédentes, **caractérisé en ce que** la distance maximale entre les deuxièmes moyens d'élastification (40, 42) au niveau d'un bord du corps absorbant (46) ou au niveau d'un bord longitudinal (48) de la section d'entrejambe (8) est plus grande dans la section dorsale (6) que dans la section ventrale (4).

8. Article pour personnes incontinentes selon une ou plusieurs des revendications précédentes, **caractérisé en ce que** les deuxièmes moyens d'élastification (40, 42) dans une zone de chevauchement (36, 38) avec le corps absorbant (7) de la zone d'entrejambe (8) sont désactivés en ce qui concerne leurs propriétés élastiques.

9. Article pour personnes incontinentes selon une ou plusieurs des revendications précédentes, **caractérisé en ce que** les deuxièmes moyens d'élastification (40, 42) conformément à la trajectoire de leur tracé en éventail sont davantage précontraints que dans une zone non disposée en éventail.

10. Article pour personnes incontinentes selon une ou plusieurs des revendications précédentes, **caractérisé en ce que** la distance (C) entre le moyen d'élastification (40, 42) de la section ventrale (4) situé le plus près de l'entrejambe et le deuxième moyen d'élastification (40, 42) correspondant de la zone dorsale (6) situé le plus près de l'entrejambe est comprise entre 250 et 420 mm.

11. Article pour personnes incontinentes selon une ou plusieurs des revendications précédentes, **caractérisé en ce que** la distance entre le deuxième moyen d'élastification (40, 42) situé le plus près de l'entrejambe et le contour (32, 34) délimitant les ouvertures de jambes de la zone (22, 26) de la section ventrale (4) et de la section dorsale (6) orientée vers l'entrejambe et les ouvertures de jambes est comprise entre 2 et 40 mm, de préférence entre 3 et 30 mm et de façon davantage préférée entre 4 et 15 mm.

12. Article pour personnes incontinentes selon une ou plusieurs des revendications précédentes, **caractérisé en ce que** l'étendue de la section ventrale (4) et de la section dorsale (6) dans la zone de couture latérale (14) dans le sens longitudinal (9) est d'au moins 100 mm, en particulier d'au moins 150 mm et en particulier entre 150 mm et 220 mm.

13. Article pour personnes incontinentes selon une ou plusieurs des revendications précédentes, **caractérisé en ce que** la distance minimale entre la section ventrale (4) et la section dorsale (6) dans le sens longitudinal (9) est comprise entre 250 et 400 mm.

14. Article pour personnes incontinentes selon une ou plusieurs des revendications précédentes, **caractérisé en ce que** l'étendue maximale de la section d'entrejambe (8) dans le sens transversal (16) est comprise entre 200 et 350 mm, en particulier entre 250 et 320 mm.

15. Article pour personnes incontinentes selon une ou plusieurs des revendications précédentes, **caractérisé en ce que** la section d'entrejambe (8) comprend un matériau de feuille arrière imperméable aux fluides (62) et un matériau de feuille supérieure (64) entre lesquels est agencé un corps absorbant (7) et **en ce que** le matériau de feuille arrière (62) et/ou le matériau de feuille supérieure (64) forment dans le sens transversal (16) une saillie (66) au-dessus du corps absorbant (7) et **en ce que** cette saillie (66) - de chaque côté du corps absorbant, c'est-à-dire à gauche et à droite - représente au moins 25 %, en particulier de 30 à 45 % et plus particulièrement de 35 à 45 % par rapport à la plus grande largeur de la section d'entrejambe (8).

16. Article pour personnes incontinentes selon une ou plusieurs des revendications précédentes, **caractérisé en ce que** la section d'entrejambe (8) présente des moyens d'élastification des jambes (82) s'étendant à l'extérieur du corps absorbant (7) le long des ouvertures de jambes (19), lesquels moyens se terminent dans le sens longitudinal (9) au moins 10 mm, en particulier au moins 20 mm avant les deuxièmes moyens d'élastification (40, 42).

**17.** Article pour personnes incontinentes selon une ou plusieurs des revendications précédentes, **caractérisé en ce que** les moyens d'élastification des jambes (82) se terminent dans le sens longitudinal (9) avant la section ventrale (4) et/ou avant la section dorsale (6).

**18.** Article pour personnes incontinentes selon une ou plusieurs des revendications précédentes, **caractérisé en ce que** la section ventrale (4) et la section dorsale (6) sont élastifiées transversalement essentiellement d'un seul tenant et de façon plane, au moins à l'extérieur du corps absorbant (7) dans le sens longitudinal (9).

**19.** Article pour personnes incontinentes selon une ou plusieurs des revendications précédentes, **caractérisé en ce que** la part de la surface de la section d'entrejambe (8) au niveau de la surface totale de l'article pour personnes incontinentes est de 25 à 55 %, et **en ce que** la section d'entrejambe (8) chevauche au moins 12 % de la surface de la section ventrale (4) et au moins 20 % de la surface de la section dorsale (6) (surface définie à chaque fois à l'état déplié comme représenté sur la figure 1).

**20.** Article pour personnes incontinentes selon une ou plusieurs des revendications précédentes, **caractérisé en ce que** la section d'entrejambe (8) chevauche entre 15 et 40 %, en particulier entre 15 et 35 % et plus particulièrement entre 15 et 25 % de la surface de la section ventrale (4) et/ou **en ce que** la section d'entrejambe (8) chevauche la section ventrale (4) sur une surface allant de 25.000 à 45.000 mm$^2$.

**21.** Article pour personnes incontinentes selon une ou plusieurs des revendications précédentes, **caractérisé en ce que** la section d'entrejambe (8) chevauche entre 20 et 40 %, en particulier entre 20 et 35 % et plus particulièrement entre 22 et 32 % de la surface de la section dorsale (6) et/ou **en ce que** la section d'entrejambe (8) chevauche la section dorsale (6) sur une surface allant de 35.000 à 65.000 mm$^2$, en particulier de 40.000 à 55.000 mm$^2$.

**22.** Article pour personnes incontinentes selon une ou plusieurs des revendications précédentes, **caractérisé en ce que** le chevauchement de la section d'entrejambe (8) est plus grand avec la zone dorsale (6) qu'avec la zone ventrale (4).

**23.** Article pour personnes incontinentes selon une ou plusieurs des revendications précédentes, **caractérisé en ce qu'**également le corps absorbant (7) chevauche entre 5 et 20 %, en particulier entre 5 et 15 % de la surface de la section ventrale (4) et/ou entre 10 et 20 %, en particulier entre 10 et 15 % de la surface de la section dorsale (6).

*Fig. 1*

*Fig. 2*

*Fig. 3*

*Fig. 4*

*Fig. 5*

*Fig. 6*

*Fig. 7*

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004052260 A1

- JP 2003126148 A